(19) Europäisches Patentamt
European Patent Office
Office européen des brevets

(11) Publication number: **0 373 623 A2**

# (12) EUROPEAN PATENT APPLICATION

(21) Application number: 89123049.2

(22) Date of filing: 13.12.89

(51) Int. Cl.5: A61K 31/16, A61K 31/165

(30) Priority: 14.12.88 US 284142

(43) Date of publication of application: 20.06.90 Bulletin 90/25

(84) Designated Contracting States: AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Bowlin, Terry L.
8466 Pond Ridge Drive
Maineville Ohio 45039(US)
Inventor: Edwards, Michael L.
12033 Deerhorn Drive
Cincinnati Ohio 45240(US)

(74) Representative: Vossius & Partner
Siebertstrasse 4 P.O. Box 86 07 67
D-8000 München 86(DE)

## (54) Pharmaceutical compositions useful in potentiating natural killer cell activity.

(57) The present invention is directed to the use of certain polyamine compounds of the following formula:

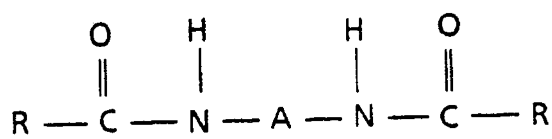

wherein A = $C_{3-20}$ alkylene or

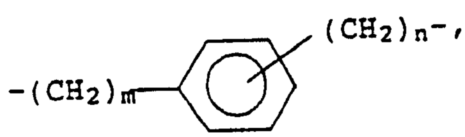

where m and n = 0-4, and
R = $C_{1-4}$ alkyl, for the preparation of a pharmaceutical composition useful in potentiating NK cell activity.

EP 0 373 623 A2

# PHARMACEUTICAL COMPOSITIONS USEFUL IN POTENTIATING NATURAL KILLER CELL ACTIVITY

The immune system is composed of two components, a humoral component and a cellular component. Natural killer (NK) cells are elements of the cellular component of the immune system. NK cells are present in most normal mammals. They exhibit cytolytic activity against a variety of cells.

The humoral component defends primarily against bacteria and toxic molecules. The humoral component consists of antibodies, or immunoglobulins. The cellular component consists of T cells, or T lymphocytes. The major effector cells of the cellular immune system are the killer T lymphocyte, macrophage and natural killer cell. The killer T cell acts mainly against virus-infected cells. Another type of killer cell closely related to the killer T cell is the NK cell. Its main targets are tumor cells and cells infected by viruses and other agents.

T cells have no detectable spontaneous activity. Rather, they are activiated upon exposure to specific antigens. In contrast, NK cells demonstrate nonspecific cytotoxic reactivity towards target cells without prior sensitization. Other factors may also activate T cells and NK cells or increase their reactivity. One of these factors, T cell growth factor, or interleukin 2 (IL2), promotes cell activitation and proliferation. Other factors may inhibit cell function, while still other factors, such as interferon, may regulate cell activity either way.

Applicants have discovered a novel method of potentiating NK cell activity which comprises the administration of certain polyamine compounds to mammals in need of potentiated NK cell activity.

The present invention relates to the use of certain polyamine compounds of the following formula:

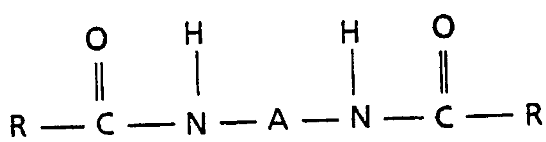

wherein A = $C_{3-20}$ alkylene or

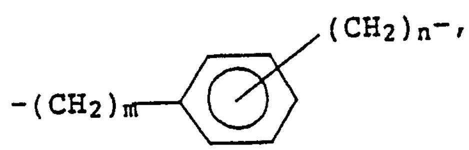

where m and n = 0-4, and
R = $C_{1-4}$ alkyl, for the preparation of a pharmaceutical composition useful in potentiating NK cell activity.

The polyamine compounds of the present invention potentiate NK cell activity. It is known that NK cells exhibit spontaneous cytolytic activity against a wide variety of cells, mainly tumor cells and cells infected by viruses and other foreign agents. It is further known NK cells play a role in the destruction of circulating tumor cells and host resistance against tumor metastasis. Thus, this evidence suggests NK cells may play an important role in immune surveillance. The ability of the polyamine compounds of the present invention to potentiate NK cells can be demonstrated as follows.

The polyamine compounds listed below were injected intraperitoneally into mice. Mice splenic lymphocytes were isolated and NK cell activity was determined against YAC-I target cells. Lymphocyte activity against YAC-I target cells was increased compared to when no compounds was given.

The polyamine compounds of the present invention can be administered in various manners to the mammal being treated to achieve the desired effect. The compounds may be administered either alone or in combination with one another, or they can be administered in the form of pharmaceutical compositions, which are well known in the art.

The compounds may be administered orally or parenterally, for example, intravenously, intraperitoneally, or subcutaneously. The amount of compound administered will vary over a wide range and can be any effective amount which will potentiate NK cell activity. Depending upon the mammal being treated, the severity of the condition being treated, the mode of administration, and the particular compound employed, the effective amount of compound administered will vary from about 1 mg/kg to 1000 mg/kg of body weight of the mammal per day.

The solid unit dosage forms can be of the conventional type. Thus, the solid form can be a capsule

which can be of the ordinary gelatin type containing a compound of the present invention and a carrier, for example, lubricant and inert fillers, such as lactose, sucrose, and corn starch. In another embodiment, the compounds are tableted with conventional tablet bases such as lactose, sucrose, and corn starch in combination with binders such as acacia, corn starch, or gelatin, disintegrating agents such as corn starch, potatoe starch, or alginic acid, and a lubricant such as stearic acid or magnesium sulfate.

For parenteral administration, the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanols and glycols, such as propylene glycol or polyethylene glycol are preferred liquid carriers, particularly for injectable solutions.

The ability of the compounds of the present invention to potentiate NK cell activity can be illustrated by the following examples.

## EXAMPLE 1

Mice were injected i.p. with the compounds listed below. The mice spleens were aseptically removed, teased apart in complete RPMI-1640, and filtered through sterile gauze to obtain single cell suspensions. Erythrocytes were lysed by suspending cells in ACK buffer (0.155 M $NH_4Cl$, 0.1 mM EDTA, and 0.01 M $KHCO_3$). The spleen cells from three mice per experimental group were pooled, washed, viability by trypan blue exclusion, and added (0.12 - 1.0 × $10^6$/0.1 ml) to a 96-well round bottom (YAC-I targets) (CoStar No. 3799) tissue culture cluster plates.

NK sensitive YAC-I lymphoma cells were used as spleen cell mediated cytotoxicity tumor cell targets. YAC-I cells ($10^6$) were labeled with 100 µCi of $^{51}Cr$ at 37°C for 1 hour. Labeled target cells were washed and added in triplicate ($10^4$/well) to the 96-well plates containing different numbers of splenic effector cells to give various E:T ratios (100/1 - 12/1) in 0.1 ml complete RPMI-1640 per well. These plates were centrifuged at 50 g for 3 minutes and incubated at 37°C for 4 hours (YAC-I). Following this incubation period, the plates were centrifuged at 300 g for 5 minutes, the supernatant fluids harvested with a supernatant collection system (Skatron, Sterlin, VA), and the released $^{51}Cr$ measured in a Beckman gamma 5500 counter. Supernatant fluids from target cells incubated alone were assayed for spontaneous release and maximum release was determined by adding 1% sodium dodecyl sulfate (SDS). Cytotoxicity, expressed as lytic units (LU), was determined as follows.

| COMPOUND | DOSE | LU |
|---|---|---|
| 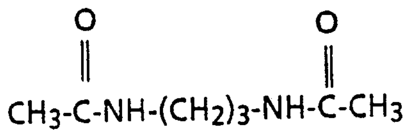 | 100 mg/kg | 5.6 |
| 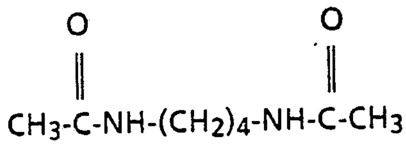 | 100 mg/kg | 5.3 |
| 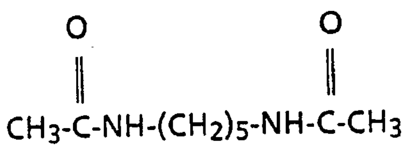 | 100 mg/kg | 5.0 |

| COMPOUND | DOSE | LU |
|---|---|---|
| 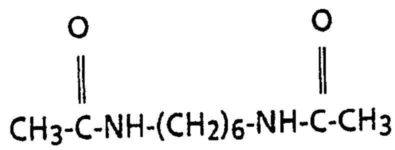 | 100 mg/kg | 5.6 |
| 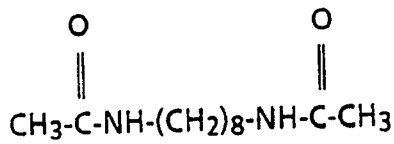 | 100 mg/kg | 5.3 |
| 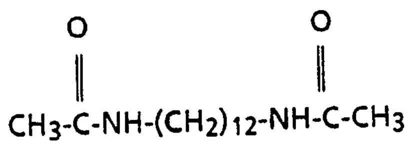 | 100 mg/kg | 10.6 |
| 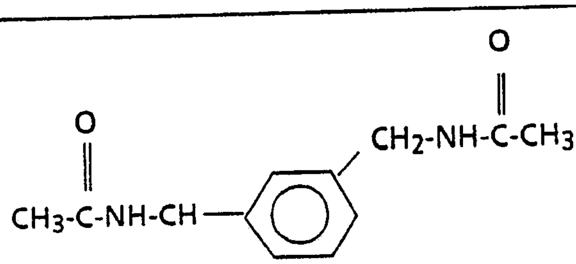 | 100 mg/kg | 6.7 |
| 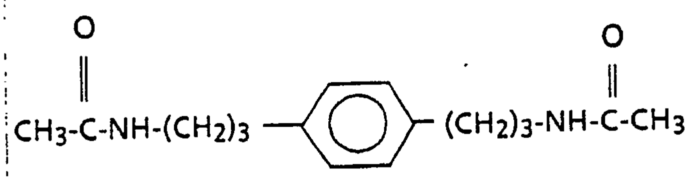 | 100 mg/kg | 4.3 |
| 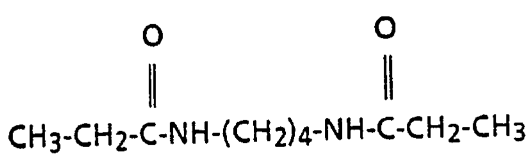 | 100 mg/kg | 5.9 |

## EXAMPLE 2

Diacetylputrescine (DAP) was administerd i.p. to mice at time 0 hours. The mice spleens were aseptically removed, teased apart in complete RPMI-1640, and filtered through sterile gauze to obtain single cell suspensions. Erythrocytes were lysed by suspending cells in ACK buffer (0.155 M $NH_4Cl$, 0.1 mM EDTA, and 0.01 M $KHCO_3$). The spleen cells from three mice per experimental group were pooled, washed, viability by trypan blue exclusion, and added (0.12 - 1.0 $\times$ $10^6$/0.1 ml) to a 96-well round bottom (YAC-I targets) (CoStar No. 3799) tissue culture cluster plates.

YAC-I lymphoma cells were used as spleen cell mediated cytotoxicity tumor cell targets. YAC-I cells

($10^6$) were labeled with 100 µCi of $^{51}Cr$ at 37° C for 1 hour. Labeled target cells were washed and added in triplicate ($10^4$/well) to the 96-well plates containing different numbers of splenic effector cells to give various E:T ratios (100/1 - 12/1) in 0.1 ml complete RPMI-1640 per well. These plates were centrifuged at 50 g for 3 minutes and incubated at 37° C for 4 hours (YAC-I). Following this incubation period, the plates were centrifuged at 300 g for 5 minutes, the supernatant fluids harvested with a supernatant collection system (Skatron, Sterlin, VA), and the released $^{51}Cr$ measured in a Beckman gamma 5500 counter. Supernatant fluids from target cells incubated alone were assayed for spontaneous release and maximum release was determined by adding 1% sodium dodecyl sulfate (SDS). Cytotoxicity, expressed as percent specific lysis, was calculated as follows:

$$\frac{\text{counts/min experimental} - \text{counts/min spontaneous}}{\text{counts/min maximum} - \text{counts/min spontaneous}} \times 100$$

Treatment with DAP increased murine NK cell activity *in vivo* as follows:

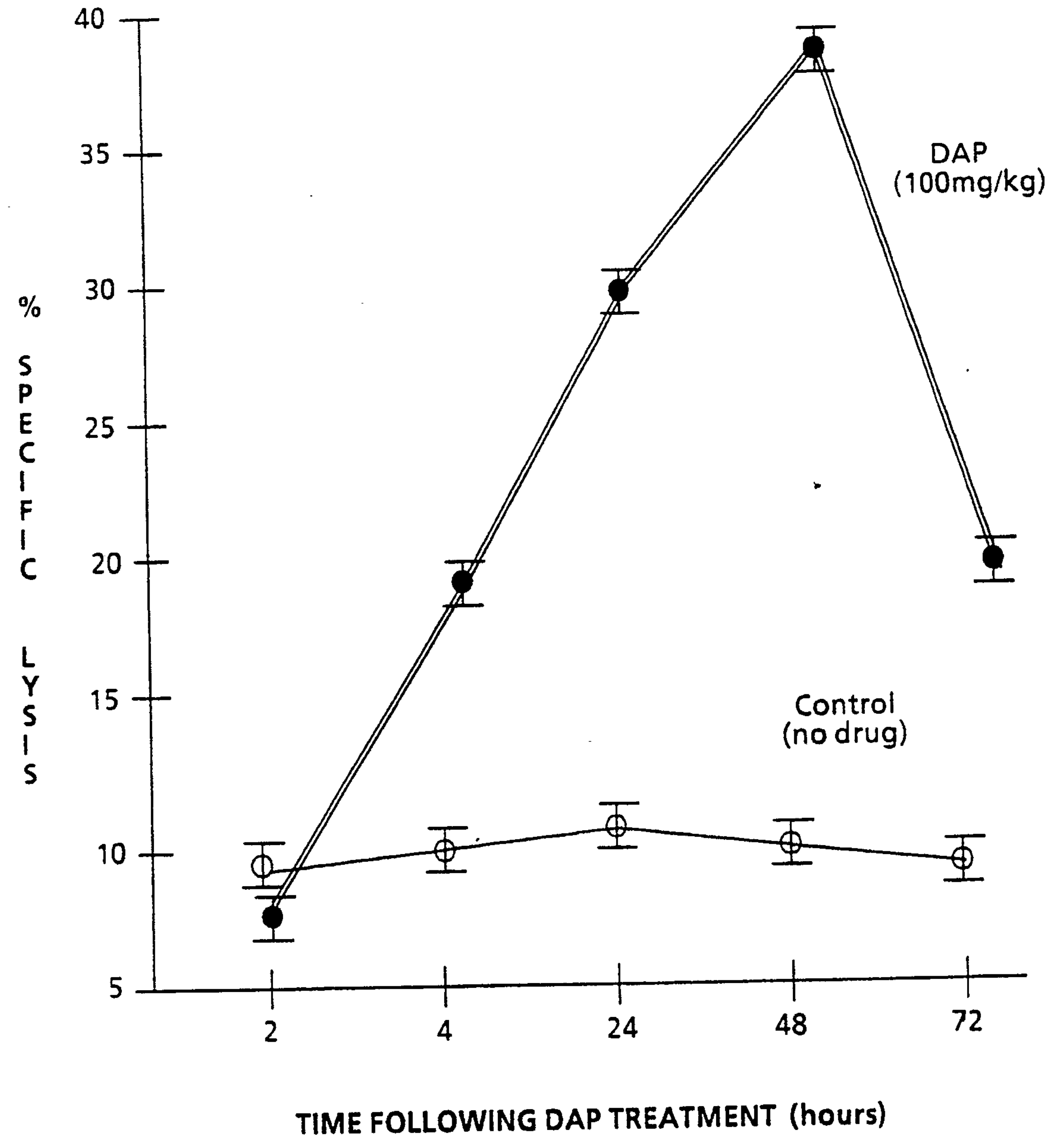

## EXAMPLE 3

Hepatic metastases were induced as follows. C57BL/6 mice were anesthetized by inhalation of pentobarbital. The mice were prepped with 70% ethanol, dried with a sterile gauze, and then, with the use of sterile scissors, a small left subcostal incision was made. The spleen was exposed, and its short gastric vessels along with the gastrosplenic ligament were cut, allowing the spleen to be brought onto the animal's abdominal wall attached to its splenic pedicle. 1 ml of a single cell suspension containing $3 \times 10^5$ tumor cells in HBSS was injected into the upper pole of the exposed extraabdominal spleen. After the injection was completed, the needle was removed, and a period of 1 minute was allowed to elapse to permit tumor cells to be flushed into the portal circulation. The splenic pedicle was clipped, the spleen was removed, and splenic pedicle was dropped i.p. The abdominal wall musculature and skin were closed in one layer. The animals were randomly allocated to their respective treatment groups and were allowed to recover. By day 3 after tumor injection, micrometastases were apparent histologically.

Each group was then treated with DAP, intraperitonealy, subcutaneously, or orally. A control group was treated with phosphate buffered saline (PBS). Metastic foci was determined and the percent reduction in metastic foci from the control group was determined with each group treated with DAP as follows:

| DAP | Metastic foci | % Reduction |
|---|---|---|
| (100 mg/kg, $d_{1-10}$) | ($x \pm$ S.E. n=8) | |
| Control | 119.8 ± 20.4[+] | ---- |
| i.p. | 40.8 ± 14.7* | 65.9 |
| s.c. | 31.0 ± 8.4* | 74.1 |
| p.o. | 27.2 ± 7.1* | 77.3 |

[+] One aminal died prior to autopsy
* $p < 0.01$

## Claims

1. The use of a polyamine compound of the formula:

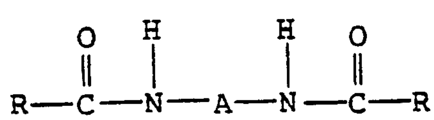

wherein A = $C_{3-20}$ alkyl or

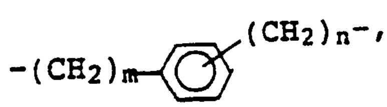

where m and n = 0-4, and
R = $C_{1-4}$ alkyl,
for the preparation of a pharmaceutical composition useful in potentiating natural killer cell activity.